# EUROPEAN PATENT APPLICATION

(11) **EP 4 332 985 A1**
(43) Date of publication of application: **06.03.2024**
(21) Application number: 22193405.2
(22) Date of filing: 01.09.2022
(51) Int. Cl.: G16H 50/20, A61B 5/00, A61K 8/02, A61Q 5/00, A45D 44/00

(54) **METHOD FOR DETERMINING A CUSTOMIZED HAIR TREATMENT FOR IMPROVING THE CONDITION OF THE STRUCTURE OF HAIR**

(71) Applicant: Henkel AG & Co. KGaA, 40589 Düsseldorf (DE)
(72) Inventor: Khadem, Behnam, 40764 Langenfeld (DE); Welzel, Jessica, 22547 Hamburg (DE); Malhotra, Aastik, 40589 Düsseldorf (DE); Koenen, Annika, 41515 Grevenbroich (DE); Grosjacques, Camille, 20255 Hamburg (DE); Bayersdoerfer, Rolf, 22589 Hamburg (DE)

(57) **Abstract**

The invention provides a method for determining a customized hair treatment for improving the condition of the structure of hair of a user. A first condition of the hair is determined prior to applying a hair treatment. Based thereon a first hair treatment selected from a database is determined. The database comprises observed effects of hair treatments on altering the condition of hair. The first hair treatment corresponds to an expected hair condition. A second condition of the hair of the user is determined after application of the first hair treatment. Based on a difference between the expected hair condition and the second condition of the hair a second hair treatment is determined. The hair condition is one among: hair damage, hair moisture.

## Description

### TECHNICAL FIELD

The invention pertains to the field of hair care and in particular to the field of maintaining the condition of hair or improving this condition by applying suitable hair treatments to hair, in particular hair care products such as shampoo, conditioner, dyes, hair bleaching agents. The invention also pertains to the field of determining hair properties in particular the effect of hair treatment on the condition of hair and to adjust hair care treatments based on observed effects of hair care treatments on hair condition. The invention provides a method that is capable of detecting the effect of previous treatments applied to hair, thereby enabling an enhanced personalized counseling for hair care, in particular advising which hair care products should be applied to hair.

### TECHNOLOGICAL BACKGROUND

Hair is a biological multicomponent fibre with a high protein content of up to 95 weight percent. Structurally, the hair cross-section can be divided into two to three distinct morphological regions, those being from outer to inner regions: the cuticle, the cortex and medulla (the medulla being present in some hairs but not all). The protein part of the cortex is made up of α-keratin helixes embedded in a proteinaceous matrix.

Cosmetic products, such as for example shampoos, conditioners, treatment masks, colorations are used to improve or change hair properties in order typically to facilitate combing, improve the smoothness of the texture of hair, increase hair gloss as well as to reduce hair breakage or to give hair a new color.

The structure of hair differs from person to person due to the many factors that can affect the inner (cortex and medulla) and outer (cuticle) structure of hair. Most often, the hair of two different people will react differently to the same hair treatment. Hair care professionals require years of experience in order to assess the potential of different hair care treatments at improving a person's hair condition and/or at achieving a desired hair property (hair color, hair gloss, hair volume, hair smoothness, hair damage which is typically an indicator of how "healthy" hair fibers are or how close to untreated and undamaged hair the hair condition is).

Even hair care professionals in salons can benefit from tools that assist them in obtaining information on the condition of hair of their clients in order to choose the most suitable hair treatments. In order to decide on products that can help improve the condition of hair (reduce damage) or maintain at a minimum the effects produced by hair damage, the SalonLab^{®} Analyzer developed by Henkel@ provides valuable information that enables users (professionals as well as non-professionals) to determine the degree of damage of their hair by measuring a cysteic acid content of their hair with infrared and near infrared sensing. The infrared and/or near-infrared spectrum is compared to a calibration model in order to assess more precisely the degree of damage of hair of the user. Further information on this approach can be found in WO2018/007353.

Despite the huge potential of such a non-intrusive optical sensing approach, the calibration model is less efficient at providing useful information about the effect of hair care products on the condition of the internal structure of hair in particular the state of the medulla and cortex. As a result, it is difficult to make accurate predictions on the effect of a hair treatment on the improvement of the condition of hair of a user without a more complete picture of the condition of the outer and inner structure of the hair of a user.

This is noticeable when trying to objectively assess the effect of a hair treatment on the full structure of the hair, including the effect on the internal structure of the hair. Some hair treatments substitute broken bonds inside hair as well as on the outside (cuticle) of hair, although only the effect on the cuticle can be easily investigated. It is difficult to estimate the exact efficiency of this improvement process without information regarding the state of the cortex and medulla and based only on the effect of the treatment on the cuticle.

A further challenge in understanding the full effect of hair treatments on hair is the influence of hair washing on the cuticle which tends to remove traces of the previous treatments applied to hair. Water tends to adsorb on the cuticle and removed traces of previous treatments which affects the ability of existing methods at accurately detecting the effect of previous hair treatments without the missing information regarding the full picture on the effect of hair treatments on the cortex and medulla of hair fibers.

For the above reasons, a method for improving the condition of the hair of a user and more particularly to determine a customized hair treatment for improving the condition of the hair of a user is sought.

### SUMMARY OF THE INVENTION

To address the above need, the invention provides a method for determining a customized hair treatment for improving the condition of the structure of hair of a user, the method comprising:
- Determining a first condition of the structure of the hair of the user prior to applying a hair treatment;
- Determining a first hair treatment based on the determined first condition, wherein the first hair treatment is selected from a database of hair treatments associated to observed effects of said hair treatments on altering the condition of the structure of the hair from different starting conditions of the structure of hair, the determined first hair treatment being associated in the database to an expected condition of the structure of the hair of the user after application of the first hair treatment to hair;
- Determining a second condition of the structure of the hair of the user after application of the first hair treatment,
- Determining a difference between the expected condition and the second condition of the structure of the hair of the user,
- Determining a second hair treatment based on the determined second condition and based on the determined difference,
Wherein the first condition, the expected condition, the second condition are at least one among: a degree of hair damage, a moisture content of hair, the presence of external chemical compounds that are on or in hair, the presence and type of chemical compounds that are on or in hair, bulk properties of constituents of hair.

This method can be viewed as part of a process in which the effect of hair treatments (in particular the application of hair care products) is assessed in a quantitative way in a user experience enabling an assessment of the efficiency of a hair treatment at achieving an improved hair condition. This method is typically implemented using a processor. The method may further be assisted by using some device for acquiring data related to the condition of hair. One such device can for example be the Salonlab^{®} Analyzer developed by Henkel@. Similarly, other devices such as a mobile phone can also be used to acquire a reconstructed hyperspectral spectrum of hair, or an image of hair allowing analysis of defects present on hair of the user.

The method of the invention proceeds iteratively by first predicting an expected change in the condition of hair from the application of a hair treatment to hair. This prediction relying on information in a database, allows the determination/selection of a hair treatment product that is expected to improve the condition of the structure of hair, for example by mitigating, alleviating or minimizing hair damage and supporting the outer and or inner structure of the hair of the user. Then, the actual effect of the application of the hair treatment to the structure of hair is determined and based on a discrepancy between the expected change and the determined change, a second hair treatment is determined. The second hair treatment is not selected solely on information available in the database but compensates for any discrepancy observed after the application of the first hair treatment, for example by selecting products having a stronger effect on mitigating hair damage if the observed second condition of the structure of the hair of the user is less improved with respect to the first determined/measured condition than expected and predicted from the database.

The determination of a first hair treatment and that of a second hair treatment is made possible by information stored in a database on the effects of different hair treatments on the condition of the structure of hair. This database can typically result from previous experiments conducted by applying hair treatments on different hair types having different initial conditions of the structure of the hair and measuring the resulting value of the condition of the structure of the hair.

Since hair has a complex structure, it is difficult to perfectly assess the full condition of the hair of a user. Two different users having very close estimated hair conditions are likely to react differently to the same hair treatment. The method of the invention provides a means to assess such differences and take corrective measures in the determination of the second hair treatment.

The term "hair treatment" may typically refer to the application of a hair product on hair such as a hair dye, a bleaching agent, a shampoo, a conditioner, a volume enhancing product, a gloss enhancing product, a waviness enhancing product. This term may also refer to other types of hair treatments such as a hair perming treatment, a hair ironing treatment, cutting the hair, adding hair extensions to the hair.

That condition can typically be a degree of hair damage that can be detected in the form of a content of cysteic acid in the hair of a user. It can also be a moisture content of hair. The first condition and the second condition may typically be determined via a measurement that relies for example on a sensing technique recording a signal from light that has interacted with hair in the infrared or near infrared spectral range and comparing that recorded signal to a calibration model. Further details on that technique are for example provided in WO2018/007353.

According to an embodiment, the method further comprises:
- obtaining a target hair condition form the user;
- determining the first hair treatment further based on the obtained target hair condition so that the first hair treatment is associated in the database to an expected condition of the structure of the hair of the user differing from the target hair condition by less than a predetermined amount.

By obtaining a target hair condition from the user, it is possible to further restrict the selection of a suitable hair treatment in order to achieve the target value provided by the user. This value can for example be a number indicative of the hair condition (indicative of a degree of hair damage of the hair of the user) on an arbitrary scale ranging from 0 (very damaged hair condition) to 100 (excellent hair, no hair damage).

According to an embodiment, the method further comprises:
- determining the second hair treatment based on a correction applied in the database to the observed effects of hair treatments on altering the condition of the structure of the hair from different starting conditions of the structure of hair, wherein the correction is estimated based on the determined difference.

The correction that is applied can advantageously rely on the hair score that is determined in the first and second determination/measurement of the condition of the structure of the hair and compared to a score of the expected change to the condition of the structure of the hair as found in the database. Based on the difference in scores, it is possible to determine the alteration of the effect of hair treatments on the specific hair of the user from the information contained in the database and simply select hair treatments with expected hair scores that are offset by an amount corresponding to the determined score difference. In this way, a user is more likely to achieve a desired target condition of the structure of hair.

According to an embodiment, the first condition, the expected condition, the second condition are determined using a method comprising:
- while exposing a hair sample of the hair of the user to at least one among light, stress, elongation or heat, recording a signal from at least one part of the light that has interacted with the hair sample or a signal from the response of the hair sample to that stress, elongation or heat;
- comparing the recorded signal with a first calibration model and determining a condition of the structure of the hair based on the comparison.

More particularly, the first calibration model can be obtained by:
- determining the condition of the structure of a plurality of calibration hair samples using a method that measures bulk properties of constituents of hair;
- exposing each of said plurality of calibration hair samples to at least one among light, stress, elongation or heat, recording a signal from at least one part of the light that has interacted with each calibration hair sample or a signal from the response of the hair sample to that stress, elongation or heat;
- establishing the first calibration model by determining a correlation between the recorded signals and the determined condition of the structure of the plurality of calibration hair samples.

Such a method is an improvement over previous methods for determining the status of hair of a user by giving access not only to the condition of the outer portion of hair (the cuticle) but instead and/or further by providing more accurate insights into the internal structure of hair (the cortex and medulla, typically on top of the information also accessible on the state of the cuticle). The above method is therefore capable of providing a more complete picture of the condition of the structure of hair fibers of a user.

The calibration method that is used is typically one that measures bulk properties of the constituents of hair. The term "bulk properties of the constituents of hair" is to be understood as referring to the internal constituents or structure of hair fibers and not only molecular vibration modes of the outer layers of hair fibers (the cuticle). Such methods rely on analytical or biophysical methods that differ from the well-known chromatography and/or High Performance Liquid Chromatography (HPLC) method of the prior art. Bulk properties give insights on components also located below the cuticle. A non-limitative list of techniques that are sensitive to the inside of the hair fibers include stress-strain measurements, fatigue measurements, elongation measurements, hair hydrophobicity or swelling behaviour, measurements of non-linear optical effects arising in hair such as birefringence of waves, attenuated total reflectance, Fourier Transform Interferometry, differential scanning calorimetry to name a few.

The calibration model is established by measuring with precision the condition of the internal structure of hair using one of such techniques on "calibration" hair samples. The same calibration hair samples are then investigated using a less sophisticated measurement technique, that can either still include one of the above techniques, or as is also particularly advantageous, a non-intrusive optical measurement using either infrared (IR), near-infrared (NIR), hyperfrequency spectrum analysis or image analysis.

The method of the invention arises from the observation that such simple and non-intrusive methods comprise traces of information regarding the condition of the internal structure of hair fibers. Such traces cannot be seen in the form of a specific absorption at certain wavelengths of the spectrum that would be documented in literature. Without the intention of being bound to theory, Applicant assumes that these signals comprise indirect effects of the internal structure of the hair that may also reflect on the cuticle in unexpected ways. Therefore, the existing IR and NIR sensing technique used in the SalonLab^{®} Analyzer developed by Henkel@ are also capable of determining the condition of the internal structure of hair using a different calibration model established on the basis of techniques that measure bulk properties of the constituents of hair.

The term "condition of the internal structure of hair" is to be understood as meaning "condition of at least the internal structure of hair" as the method of the invention can be sensitive to both the internal structure of hair and the outer structure of hair, thereby providing a more complete information regarding the overall condition of the structure of the hair of a user.

According to an embodiment, the method that measures bulk properties of constituents of hair comprises at least one among: differential scanning calorimetry applied to hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

The above list of techniques give access to the internal structure of hair fibers and thereby further contribute to the establishment of a more sophisticated calibration model than prior art methods.

According to an embodiment, the method further comprises:
- obtaining a second calibration model that establishes a correlation between a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat.

The second calibration model typically relies on information that is already used in prior art in the Salonlab^{®} Analyzer device developed by Henkel@. It is a calibration model conceived to detect the degree of damage of hair in particular through a determination of a content of cysteic acid in hair, and more particular in the cuticle of hair.

According to an embodiment, the signal recorded while exposing the hair sample of the hair to at least one among light, stress, elongation or heat is compared to the second calibration model to determine a degree of hair damage of the hair sample.

Such a comparison provides the possibility to distinguish between the contribution of the cuticle to the condition of hair fibers and the contribution of the cortex and medulla to the condition of hair fibers. A subtraction of the signal recorded using both calibration models provides insights into this specific contribution of the internal part of the structure of the hair fiber to the condition of hair. This internal part typically comprises stronger markers of the previous hair treatments applied to hair which provides a means to determine how effective a hair treatment is at improving an overall condition of hair of a user. This constitutes a valuable tool for salon professionals and individuals alike in understanding the effect of hair treatments on the hair of users and adapt these treatments to the real and quantitatively assessed condition of the hair.

According to an embodiment, the method further comprises:
- determining a first value of the condition of the structure of hair prior to applying a hair treatment to the hair;
- determining a second value of the condition of the structure of hair after application of the hair treatment;
- comparing the difference between the second value of the condition of the structure of hair and the first value of the condition of the structure of hair;
- determining, based on said comparison, a specific contribution of the application of the hair treatment to the condition of the structure of hair.

Such an approach is one way to determine the specific contribution of the application of a hair treatment to the condition of hair of a user.

According to another embodiment, the method further comprises:
- determining a relationship between values of the condition of the structure of hair obtained using the first calibration model and values of the degree of hair damage obtained using the second calibration model;
- determining a value of the condition of the structure of hair and a value of the degree of hair damage after application of the hair treatment;
- using the determined relationship to transform the value of the degree of hair damage into a corresponding equivalent value of the condition of the structure of hair;
- subtracting from the determined value of the condition of the structure of hair the corresponding equivalent value to estimate the additional contribution of the application of the hair treatment on the value of the condition of the structure of hair.

Such an approach is an alternative to the determination of the specific contribution of the application of a hair treatment to the condition of the structure of the hair of a user.

According to an embodiment, the method further comprises:
- obtaining a user input regarding previous hair treatments applied to hair;
- adjusting the first hair treatment and the second hair treatment further based on the obtained user input.

By gathering input from the user, it is possible to apply corrective steps to the determined hair score by offsetting the determined hair score from the first condition of the structure of the hair of the user by an amount corresponding to the contribution of these past hair treatments. The contribution of hair treatments to the condition of the structure of the hair and the hair score may typically be stored in the database. In this way, the first hair treatment recommendation can be more accurate and better predict the effect of its application on the condition of the structure of the hair of a user.

According to an embodiment, the method further comprises:
- obtaining a user input regarding a desired target hair condition;
- adjusting the first hair treatment and the second hair treatment further based on the obtained user input.

According to an embodiment, the method further comprises:
- Determining, based on the determined first condition, a customized first hair treatment comprising a mixture of hair treatment products, wherein the database comprises information relating to each hair treatment product of the mixture;
- Determining, based on the determined second condition and on the determined difference, a customized second treatment comprising a mixture of hair treatment products, wherein the database comprises information relating to each hair treatment product of the mixture.

The method of the invention can be used to generate combinations, mixtures of chemical compositions that are tailor-made for a user, thereby providing effects that cannot be reached using only products available on the market. The determination of the ratios of such mixtures can be made based on the contribution of each individual product to the expected condition of the structure of the hair, and the determined/measured value of the condition of the structure of the hair of the user, as well as the target condition.

The invention also pertains to a computer program product comprising program instructions configured to implement a method for determining a customized hair treatment for improving the condition of a structure of hair of a user, the method comprising:
- Determining a first condition of the structure of hair of the user prior to applying a hair treatment;
- Determining a first hair treatment based on the determined first condition, wherein the first hair treatment is selected from a database of hair treatments associated to observed effects of said hair treatments on altering the condition of the structure of hair from different starting conditions of the structure of hair, the determined first hair treatment being associated in the database to an expected condition of the structure of the hair of the user after application of the first hair treatment to hair;
- Determining a second condition of the hair of the user after application of the first hair treatment,
- Determining a difference between the expected hair condition and the second condition of the structure of the hair of the user,
- Determining a second hair treatment based on the determined second condition and based on the determined difference,
Wherein the first condition, the expected condition, the second condition are at least one among: a degree of hair damage, a moisture content of hair, the presence of external chemical compounds that are on or in hair, the presence and type of chemical compounds that are on or in hair, bulk properties of constituents of hair.

The computer program implements the method of the invention, typically with the help of a processor.

In other words, the invention also pertains to a non-transitory computer-readable storage medium having stored thereon program instructions configured to implement the steps of a method for determining a customized hair treatment for improving the condition of a structure of hair of a user, the method comprising:
- Determining a first condition of the structure of hair of the user prior to applying a hair treatment;
- Determining a first hair treatment based on the determined first condition, wherein the first hair treatment is selected from a database of hair treatments associated to observed effects of said hair treatments on altering the condition of the structure of hair from different starting conditions of the structure of hair, the determined first hair treatment being associated in the database to an expected condition of the structure of the hair of the user after application of the first hair treatment to hair;
- Determining a second condition of the hair of the user after application of the first hair treatment,
- Determining a difference between the expected hair condition and the second condition of the structure of the hair of the user,
- Determining a second hair treatment based on the determined second condition and based on the determined difference,
Wherein the first condition, the expected condition, the second condition are at least one among: a degree of hair damage, a moisture content of hair, the presence of external chemical compounds that are on or in hair, the presence and type of chemical compounds that are on or in hair, bulk properties of constituents of hair.

### BRIEF DESRIPTION OF THE DRAWINGS

The present disclosure will hereinafter be described in conjunction with the following drawing figures, wherein like numerals denote like elements, and:
Fig. 1 is a flowchart illustrating steps of the method according to an embodiment;
Fig. 2 is a flowchart illustrating an aspect of an embodiment;
Fig. 3 is a flowchart illustrating a further aspect of an embodiment;
Fig. 4, 5 and 6 are schematic illustrations of a user interface implementing aspects of an embodiment;
Fig. 7 is a schematic illustration of the implementation of a method according to an embodiment on the hair of a user using a device.

### DETAILED DESCRIPTION

The invention provides a method for determining a hair treatment that is specific to a user and that is capable of improving the condition of the structure of the hair of a user. This method relies on a approach that assesses the effect of hair treatments on the hair of a user, further assisted by technologies enabling the determination with sufficient accuracy of the condition of the structure of the hair of a user.

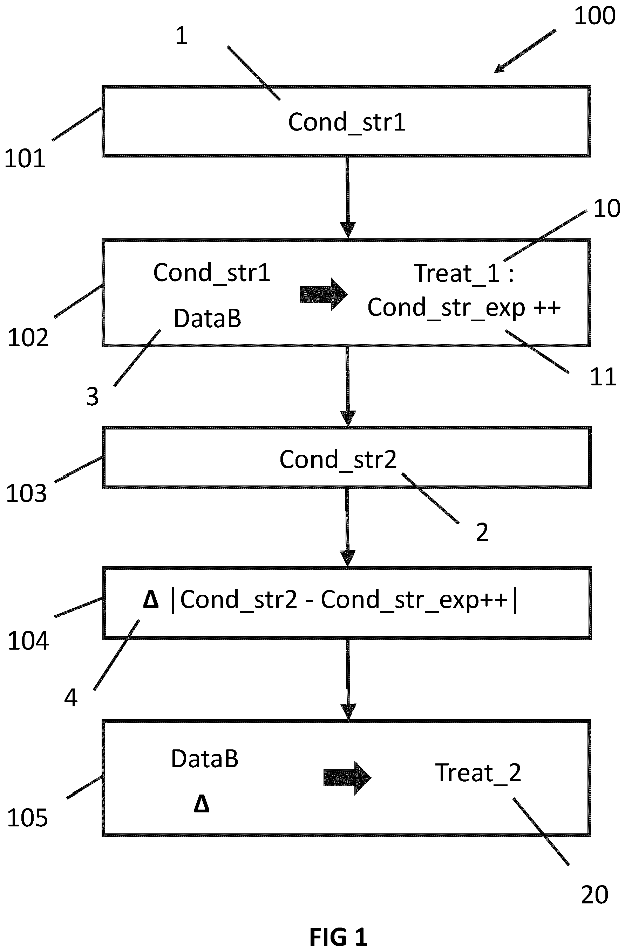

Figure 1 provides a flowchart that illustrates steps of an embodiment of the method 100 of the invention.

A first condition 1 of the structure of the hair of a user is determined 101. This determination can typically be made by measuring a signal that is recorded using optical means. The SalonLab^{®} Analyzer developed by Henkel@ is one device enable the recording of such a signal.

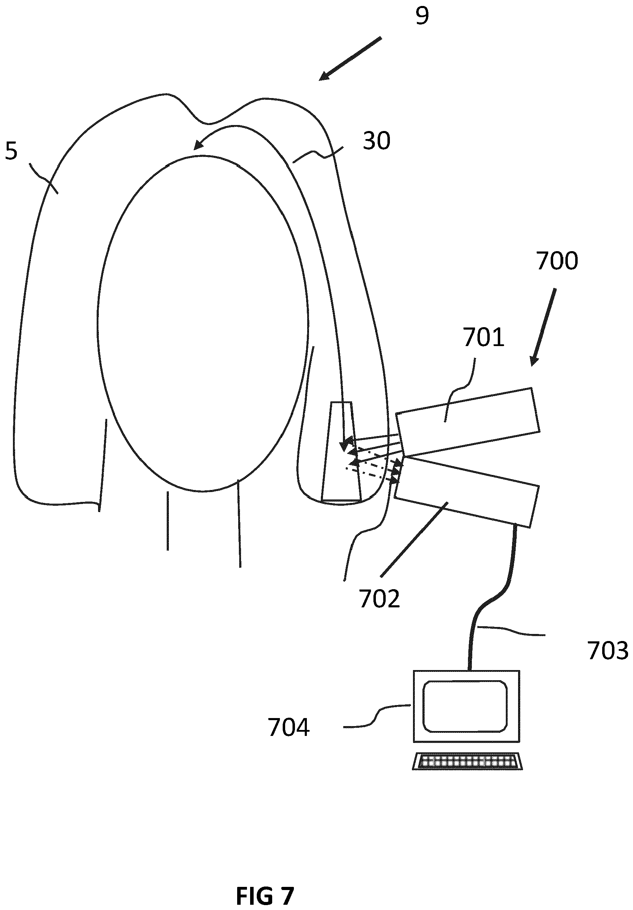

Typically the measurement can be implemented so that hair of a user and more particularly a hair sample is subjected to a stimulus and the response of hair to that stimulus is measured with a detector. A handheld device that uses an optical signal to determine the condition of the structure of hair of the user can be used for that purpose. Figure 7 commented hereunder in more detail provides one example of such a device. The device comprises a light emitter that emits light for example in the infrared and/or near infrared spectral ranges. The infrared spectral range typically comprises wavelengths between 700 nm and 1 mm. The near infrared spectral range typically comprises wavelengths between 700 nm and 2500 nm. Infrared and near infrared are particularly advantageous spectral ranges for observation of physical objects because they can be analyzed in a non-intrusive, non-destructive way and provide insights into vibrational modes of molecules forming the object that is investigated.

Vibrational modes of molecules can in particular be used to measure the degree of hair damage via the amount of cysteic acid that is present in hair. Indeed, cysteic acid is a marker of broken dissulfide bonds (S-S) in hair and an accurate indicator of the damage state of hair.

Apart from infrared or near-infrared sensing, the device may rely on other optical sensing technologies such a FTIR (Fourier Transform Interferometry), ATR (attenuated total reflectance), hyperspectral imaging (for example either by using a hyperspectral camera or by reconstructing a hyperspectral signal from several narrow band images of hair acquired under different illuminates for example those that can be generated with a portable phone as described for example in WO2018/228859. It is also possible to acquire information regarding the condition of the hair of the user using an imaging device in the visible spectral range. Such image analysis tools can detect defects on hairs provided the image has a high enough resolution. Detection of the defects in the image can be determined based on a detection algorithm trained using a plurality of images of hair of known damage state using a calibration model.

The method 100 further accesses information contained in a database 3 to determine 102 a first hair treatment 10 which according to the database 3 is expected to improve the condition of the structure of the hair of the user from the first condition 1 to an expected condition 11. This information is assessed from the database 3 based on the value of the determined first condition 1. The determination 102 may further also select the first hair treatment 10 based on additional input provided by the user, such as previous hair treatments that the user applied to hair in the past few days or weeks or based on a target value for the condition of the structure of the hair of the user input by the user.

The term "condition of the structure of the hair" typically refers to the degree of hair damage, the presence of external chemical compounds that are on or in hair, the presence and type of chemical compounds that are on or in hair, the degree of hair moisture. Other parameters might also be included such as a glossiness value of hair, a value indicative of hair volume, a value indicative of hair waviness.

After application of the first hair treatment 10, a second condition 2 of the structure of the hair of the user is determined 103. The same technique as that described above can typically be used.

The determined second condition 2 is compared 104 to the expected condition 11, thereby leading to the estimation of a difference 4 between both values.

Using information stored in the database 3 and the difference 4 determined as explained above, the method finally determines 105 a second hair treatment 20 which takes into consideration the discrepancy between information contained in the database and the determined/measured condition of the structure of the hair of the user.

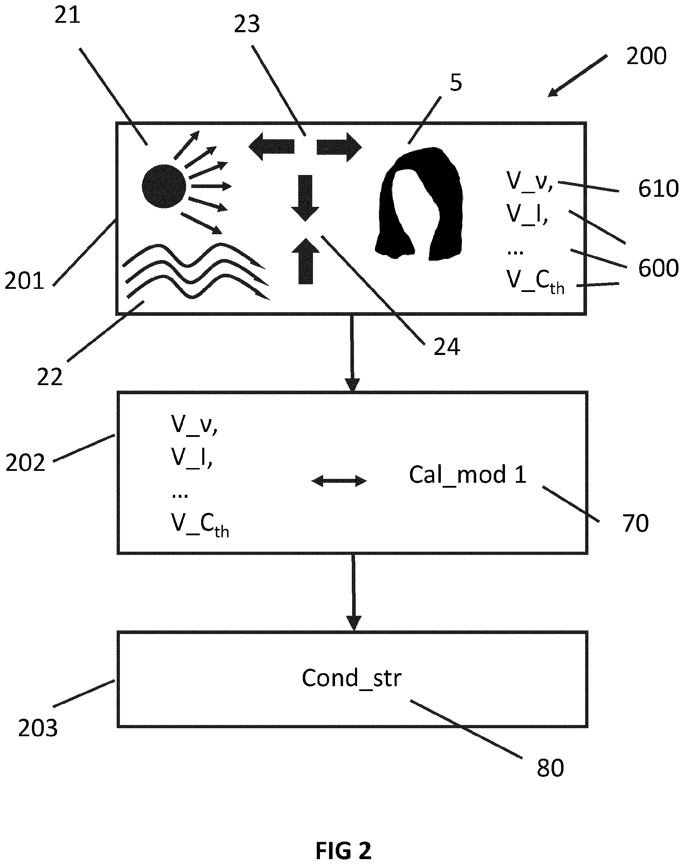

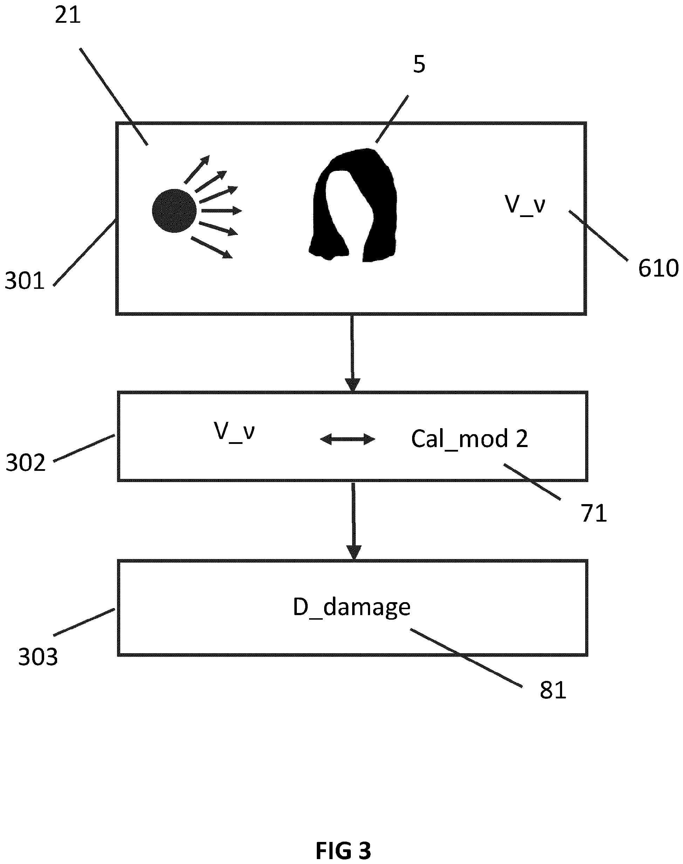

Figures 2 and 3 provide flowcharts describing how the condition of the structure of the hair of the user can be determined.

Method 200 such as schematically illustrated on the flowchart of figure 2 can be used to determine a value for the condition of the structure of the hair of a user. More particularly, this method 200 is capable of accessing not only the condition of the cuticle, but also of the cortex and/or medulla of the hair. This method 200 typically comprises a step of recording 201 a signal 610 from at least one part of the light 21 that has interacted with the hair 5 while exposing hair 5 to light 21. As seen on figure 2, other means of recording a signal can be implemented such as for example exposing hair 5 to one among stress 24 (such as mechanical stress, shear stress, compressive stress, torsional stress), elongation 23 or heat 22. The response of hair 5 to such an input is then recorded in the form of a signal 600. The heat response of hair 5 can be recorded in a differential scanning calorimetry measurement. In such an experimental setup, hair samples are exposed to heat in order to determine the amount of heat required to increase the temperature of the hair sample. This gives access to the heat capacitance of the hair sample which can provide insights into the condition of the internal structure of the hair sample.

The most preferred embodiment consists in recording a signal 610 corresponding to light that has interacted with hair 5, in particular infrared and/or near-infrared light.

Then, the recorded signal 600, 610 is compared 202 to a first calibration model 70. The calibration model 70 is for example a chart or mathematical function that provides a correspondence between a value of the recorded signal 600, 610 and a value of the condition of the internal structure 80 of the hair 5.

Based on this comparison, the condition of the internal structure 80 of the hair 5 is determined 203 by identifying the value within the calibration model 70 that represents a closest match to the value of the recorded signal 600, 610.

One strength of the method of the invention relies in the ability to determine this value of the internal structure 80 of the hair 5 even with sensing techniques that are not in principle capable to directly assess such an internal structure (that is to say, the structure of the hair 5 below the cuticle). One aspect of the invention relies in the establishment of the first calibration model 70 used in the method 200 of the invention.

The first calibration model 70 is established by measuring the condition of the internal structure 80 of calibration hair samples. The value of the condition of the internal structure 80 of these calibration hair samples is known either because the hair was exposed to an event that caused the condition of the internal structure 80 to reach a predetermined value, or this value is measured using a method that measures bulk properties of constituents of hair 5. Such a method is typically one which is sensitive to the internal structure of the hair 5 below the cuticle and can involve one among: differential scanning calorimetry applied to hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

The same calibration hair samples are then investigated using a different method. Advantageously, this other method is non-intrusive, non-destructive and relies on optical sensing means. Preferentially the other method is an infrared and/or near-infrared sensing technique that measures an infrared and/or near-infrared spectrum of light that has interacted with hair while exposing hair to such light. Alternatively, the type of signals recorded that were mentioned above (exposing the hair sample of hair 5 to one among stress 24 such as mechanical stress, shear stress, compressive stress, torsional stress, elongation 23 or heat 22 and recording the response of the hair to such stimulus) may also be used in this other method.

Since the calibration hair sample necessarily has the same value for the condition of the internal structure 80 thereof when measured using these two different methods, both values determined using the two different measurement techniques can be compared to establish a calibration curve.

According to a preferred embodiment, the values of the condition of the internal structure 80 of the calibration hair samples is determined using differential scanning calorimetry. Then the condition of the internal structure 80 of the calibration hair samples is measured using an infrared and/or near-infrared sensing technique such as one already known in connection with the SalonLab^{®} Analyzer developed by Henkel@.

The correspondence established between the values acquired using the optical detection means and the differential scanning calorimetry measurements provides a way to determine the condition of the internal structure 80 of hair samples using a simple non-intrusive and non-destructive method on any hair samples of any user. Another advantage of the use of an optical detection method is that it is faster to implement than other mechanical methods or methods relying on differential scanning calorimetry.

The method 200 of the invention enables a more complete and more objective and accurate quantitative determination of the state of the hair 5 of a user. This can be used in particular to determine the effect of hair treatments on the hair 5 of a user, and further used to recommend more customized and more suitable hair treatments that take into consideration the measured effect of the treatments on the specifical hair 5 of users.

Several approaches may be implemented in order to extract from the method 200 presented above, the specific contribution of a hair treatment on the condition of hair 5 of a user.

One approach consists in taking a first measurement of the value of the condition of the internal structure 80 of the hair 5 of a user before a hair treatment is applied to said hair 5. Then, another measurement of the value of the condition of the internal structure 80 of the hair 5 of the user is made after application of the hair treatment to said hair 5. Since both measurement techniques are sensitive to the internal structure of the hair 5 of the user, the value of the first measurement can be subtracted from the value of the second measurement in order to extract the contribution to the value of the condition of the internal structure 8 of the hair 5 of the user attributable only to the hair treatment itself.

The term "hair treatment" typically refers to at least one among: application of a hair product such as a hair dye, a hair bleaching agent, a shampoo, a conditioner, a hair volume enhancing agent, a hair gloss enhancing agent, a hair perming treatment, a hair ironing treatment, cutting the hair, adding hair extensions to the hair 5 of the user.

Another approach consists in using two different calibration models in processing the same measurement of the value of the condition of the internal structure 80 of hair 5 of a user.

Figure 3 provides another schematic flowchart which represents steps of a method used in determining the degree of hair damage 81 using a second calibration model 71. The process represented on figure 3 corresponds to the state of the art already known and described for example in WO2018/007353. The process of figure 3 also comprises a step of recording 301 a signal corresponding to light 21 that has interacted with hair 5 of a user. The recorded signal 610 is compared 302 to a second calibration model 71. Based on this comparison, the degree of hair damage 81 is determined 303.

The second calibration model 71 is established using the approach described for example in WO2018/007353. Calibration hair samples are investigated using chromatography or HPLC (High Performance Liquid Chromatography) in order to determine their degree of hair damage which is also proportional to the cysteic acid content of the calibration hair samples. The same content of cysteic acid is determined using infrared and/or near-infrared sensing of the calibration hair samples. A correlation is established between the optical signal that is acquired using infrared and/or near-infrared sensing and the signal determined using HPLC or chromatography to build the second calibration model.

As opposed to the first calibration model 70, the second calibration model 71 is sensitive essentially to the degree of damage 81 of the cuticle of the hair 5. This provides a way to extract the contribution of a hair treatment to a value of the condition of the internal structure 80 of hair 5 from a single optical measurement of the signal 610 recorded from light 21 that has interacted with hair 5 or from any other measurement acquiring signals 600 as described above. It has been observed that there is a substantially linear dependency between the evolution of the degree of damage of hair 5 detectable using the second calibration model 71 and the evolution of the condition of the internal structure 80 of hair 5 detectable using the first calibration model 70. Establishing this correlation, it is possible to identify the specific contribution of the hair treatment to the value of the condition of the internal structure 80 of hair 5 by determining the value of the condition of the internal structure 80 of hair 5 using the first calibration model 70, determining the value of the degree of hair damage using the second calibration model 71 , using the correlation to transform the value of the degree of hair damage into a corresponding equivalent value of the condition of the internal structure of hair and subtracting from the determined value of the condition of the internal structure 80 of hair 5 the corresponding equivalent value.

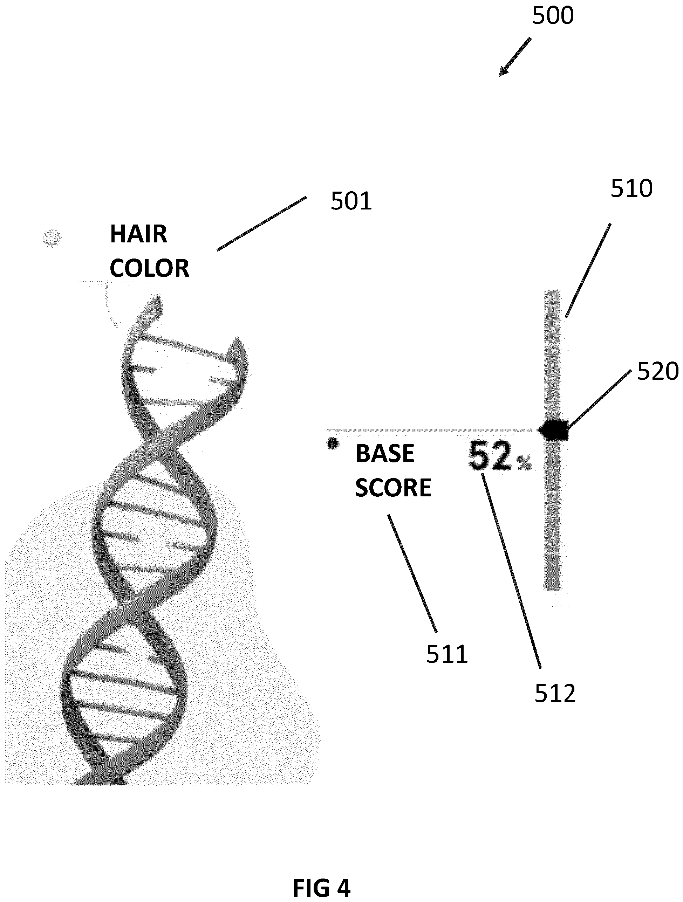

The method 100 can be implemented by a computer program that can be run on any terminal in the form of an app or any other user interface as schematically represented on figures 4, 5 and 6. Figures 4-6 further illustrate how the method 100 of the invention may display measurements in a format that is understandable to a user.

As seen on figure 4, the user interface 500 can indicate information such as the hair color 501 of the user and provide a scale 510 on which values of the condition of hair are represented from very poor (at the lower end of the scale 510) to very good (at the upper end of the scale 510). On figure 4, the scale 510 is subdivided into 5 different qualitative ranges. Each value of the condition of hair is associated with a score provided as a number between 0 (very poor condition) to 100 (very good condition). On Figure 4, a base score 511 is provided. Base score 511 indicates that the measurement corresponds to an initial condition of the internal structure of hair 5 of the user, prior to application of any hair treatment.

Naturally, users already exposed their hair to some hair treatment, be it by exposure to UV light by being exposed to sunlight, temperature changes, air pollution, previous application of hair care products. The base score 511 is an approximation of an untreated, non-product treated hair condition, even though in reality the past history of the hair 5 of the user already includes use of hair products which is detectable in the achieved score 542 with the method 100 of the invention.

The numerical value 512 of that base score is provided so as the position 520 of the based score on the scale 510, in particular within one of the five category types. The scale 510 is an arbitrary unit scale that is representative of the degree of hair damage of the hair 5 of the user (which can be viewed as an indication of how "healthy" hair is). This hair damage may reflect either only the damage as measured at the cuticle of hair 5 using the second calibration model 71 or the more complete hair damage as measured using the first calibration model 70.

User interface 500 may further comprise a questionnaire that collects further information from a user, in particular information regarding past hair treatments in order to adjust the value of the base score 511.

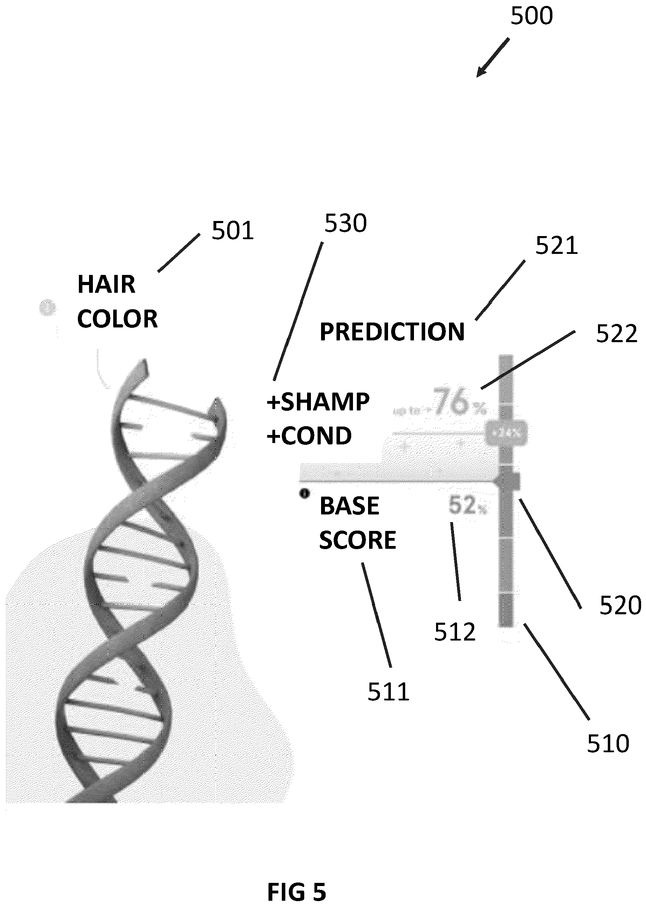

As seen on figure 5, the user interface 500 may further comprise a recommendation of a hair treatment based on a prediction 521 of the achievable hair score 522 when applying a hair treatment 530 such as a shampoo and/or a conditioner to the hair 5 of the user. Such a prediction may typically be possible based on a database that associates hair treatments to a quantified effect of the hair treatment on improving a condition of hair after application of said hair treatment having a certain initial condition of the internal structure of hair 5.

The prediction 521 may not fully reflect the true hair score that a user will achieve when applying the hair treatment 530, since the bae score 511 may already include the effect of previous known or even unknown hair treatment to which hair 5 of the user was exposed. Furthermore, hair 5 of users being dependent on many different factors, it is likely that two different users having a similar base score 511 will in fact react differently when the same hair treatment 530 is applied to their hair.

The method 100 of the invention can assist in determining such differences and apply corrective measures to iteratively make better and better recommendations for future hair treatments 530 based on a measured evolution of the hair score.

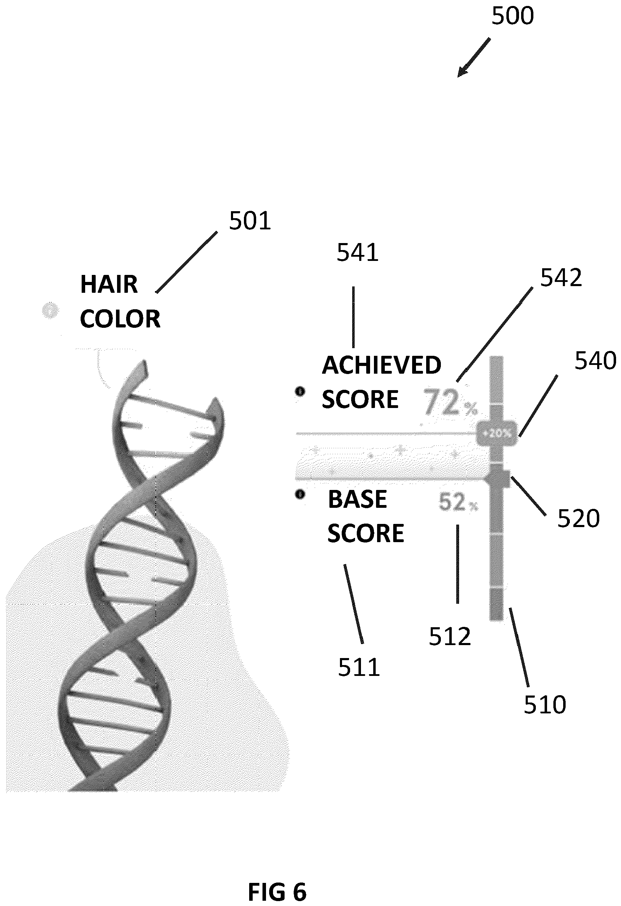

Figure 6 schematically illustrates the achieved score 541 measured on the hair 5 of a user after application of the hair treatment 530 recommended in figure 5. One observes that the achieve hair score 542 has a value that is slightly lower than the one predicted in figure 5. This difference can be used to determine facts regarding the effect of past treatments on the base score 511. Figure 6 is further evidence that the method 100 of the invention is capable of quantifying the effect 540 of the application of a hair treatment 530 on the value of the condition 80 of an internal structure of hair 5.

Based on the information that is available from the application of the method 100 of the invention, it is possible to adjust the hair treatment 530 recommendations made to a user and determine which products or hair treatments 530 have the strongest and most desired effect of the condition 80 of the internal structure of their hair 5.

Figure 7 provides a schematic representation of a device 700 usable in implementing parts of the method 100 according to the invention and methods 200, 300 mentioned above. Hair of a user and more particularly a hair sample 30 is subjected to a stimulus and the response of hair to that stimulus is measured with a detector 702. In the example represented on figure 7 the device 700 is a handheld device that uses an optical signal to determine the condition of the internal structure of hair 5 of the user. The device 700 comprises a light emitter 701 that emits light for example in the infrared and/or near infrared spectral ranges.

The device 700 may also be replaced by a mobile phone and have different shapes that are not represented on the figures. Some shapes of the device 700 can for example be more suitable to access the root length and tips of hair and differ from the V or U-shaped device represented on figure 7.

Figure 7 further illustrates that the device 700 may communicate either wirelessly of in a wired 703 manner with a remote data processing device such as a computer 704, a cloud service or server. The device 700 may also comprise computing means in the device itself.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the various embodiments in any way. Rather, the foregoing detailed description will provide those skilled in the art with a convenient road map for implementing an exemplary embodiment as contemplated herein. It being understood that various changes may be made in the function and arrangement of elements described in an exemplary embodiment without departing from the scope of the various embodiments as set forth in the appended claims.

## Claims

1. A method for determining a customized hair treatment for improving the condition of the structure of hair of a user, the method comprising:
- Determining a first condition of the structure of the hair of the user prior to applying a hair treatment;
- Determining a first hair treatment based on the determined first condition, wherein the first hair treatment is selected from a database of hair treatments associated to observed effects of said hair treatments on altering the condition of the structure of the hair from different starting conditions of the structure of hair, the determined first hair treatment being associated in the database to an expected condition of the structure of the hair of the user after application of the first hair treatment to hair;
- Determining a second condition of the structure of the hair of the user after application of the first hair treatment,
- Determining a difference between the expected condition and the second condition of the structure of the hair of the user,
- Determining a second hair treatment based on the determined second condition and based on the determined difference,
Wherein the first condition, the expected condition, the second condition are at least one among: a degree of hair damage, a moisture content of hair, the presence of external chemical compounds that are on or in hair, the presence and type of chemical compounds that are on or in hair, bulk properties of constituents of hair.

2. The method of claim 1, further comprising:
- obtaining a target hair condition form the user;
- determining the first hair treatment further based on the obtained target hair condition so that the first hair treatment is associated in the database to an expected condition of the structure of the hair of the user differing from the target hair condition by less than a predetermined amount.

3. The method of claim 1 or claim 2, further comprising:
- determining the second hair treatment based on a correction applied in the database to the observed effects of hair treatments on altering the condition of the structure of the hair from different starting conditions of the structure of hair, wherein the correction is estimated based on the determined difference.

4. The method according to any one of the preceding claims, wherein the first condition, the expected condition, the second condition are determined using a method comprising:
- while exposing a hair sample of the hair of the user to at least one among light, stress, elongation or heat, recording a signal from at least one part of the light that has interacted with the hair sample or a signal from the response of the hair sample to that stress, elongation or heat;
- comparing the recorded signal with a first calibration model and determining a condition of the structure of the hair based on the comparison.

5. The method according to claim 5, wherein the first calibration model is obtained by:
- determining the condition of the structure of a plurality of calibration hair samples using a method that measures bulk properties of constituents of hair;
- exposing each of said plurality of calibration hair samples to at least one among light, stress, elongation or heat, recording a signal from at least one part of the light that has interacted with each calibration hair sample or a signal from the response of the hair sample to that stress, elongation or heat;
- establishing the first calibration model by determining a correlation between the recorded signals and the determined condition of the structure of the plurality of calibration hair samples.

6. The method according to claim 5 wherein the method that measures bulk properties of constituents of hair comprises at least one among: differential scanning calorimetry applied to hair, electron microscopy of hair, stress-strain measurements, fatigue measurements, elongation measurements, hair hydrophobicity or swelling detection, measurements of non-linear optical effects arising in hair such as birefringence of waves, attenuated total reflectance, Fourier Transform Interferometry.

7. The method according to any one of claims 4 to 6, wherein the method further comprises:
- obtaining a second calibration model that establishes a correlation between a determined degree of hair damage of the calibration hair samples and signals recorded from at least one part of the light that has interacted with calibration hair samples or signals corresponding to a response of hair to stress, elongation or heat while exposing calibration hair samples of the hair to that light, stress, elongation or heat.

8. The method according to claim 7, wherein the signal recorded while exposing the hair sample of the hair to at least one among light, stress, elongation or heat is compared to the second calibration model to determine a degree of hair damage of the hair sample.

9. The method according to any one of claims 7 or 8, further comprising:
- determining a first value of the condition of the structure of hair prior to applying a hair treatment to the hair;
- determining a second value of the condition of the structure of hair after application of the hair treatment;
- comparing the difference between the second value of the condition of the structure of hair and the first value of the condition of the structure of hair;
- determining, based on said comparison, a specific contribution of the application of the hair treatment to the condition of the structure of hair.

10. The method according to claim 7, further comprising:
- determining a relationship between values of the condition of the structure of hair obtained using the first calibration model and values of the degree of hair damage obtained using the second calibration model;
- determining a value of the condition of the structure of hair and a value of the degree of hair damage after application of the hair treatment;
- using the determined relationship to transform the value of the degree of hair damage into a corresponding equivalent value of the condition of the structure of hair;
- subtracting from the determined value of the condition of the structure of hair the corresponding equivalent value to estimate the additional contribution of the application of the hair treatment on the value of the condition of the structure of hair.

11. The method according to any one of the preceding claims, wherein the method further comprises:
- obtaining a user input regarding previous hair treatments applied to hair;
- adjusting the first hair treatment and the second hair treatment further based on the obtained user input.

12. The method according to any one of the preceding claims, wherein the method further comprises:
- obtaining a user input regarding a desired target hair condition;
- adjusting the first hair treatment and the second hair treatment further based on the obtained user input.

13. The method according to any one of the preceding claims, further comprising:
- Determining, based on the determined first condition, a customized first hair treatment comprising a mixture of hair treatment products, wherein the database comprises information relating to each hair treatment product of the mixture;
- Determining, based on the determined second condition and on the determined difference, a customized second treatment comprising a mixture of hair treatment products, wherein the database comprises information relating to each hair treatment product of the mixture.

14. Computer program product comprising program instructions configured to implement a method for determining a customized hair treatment for improving the condition of a structure of hair of a user, the method comprising:
- Determining a first condition of the structure of hair of the user prior to applying a hair treatment;
- Determining a first hair treatment based on the determined first condition, wherein the first hair treatment is selected from a database of hair treatments associated to observed effects of said hair treatments on altering the condition of the structure of hair from different starting conditions of the structure of hair, the determined first hair treatment being associated in the database to an expected condition for the structure of the hair of the user after application of the first hair treatment to hair;
- Determining a second condition of the hair of the user after application of the first hair treatment,
- Determining a difference between the expected hair condition and the second condition of the structure of the hair of the user,
- Determining a second hair treatment based on the determined second condition and based on the determined difference,
Wherein the first condition, the expected condition, the second condition are at least one among: a degree of hair damage, a moisture content of hair, the presence of external chemical compounds that are on or in hair, the presence and type of chemical compounds that are on or in hair, bulk properties of constituents of hair.
